# EUROPEAN PATENT SPECIFICATION

(11) **EP 1 064 956 B1**
(45) Date of publication and mention of the grant of the patent: **14.09.2005**
(21) Application number: 00401824.8
(22) Date of filing: 26.06.2000
(51) Int. Cl.: A61L 9/01, A61L 15/46, A61L 9/013, A61L 15/38

(54) **Deodorizing sanitary articles**
Desodorisierende Hygieneartikel
Articles hygiéniques désodorisants

(30) Priority: 24.06.1999 JP 17818299
(43) Date of publication of application: 03.01.2001
(73) Proprietor: Takasago International Corporation, Tokyo (JP)
(72) Inventor: Mishima, Yasutaka, TAKASAGO INTER. CORP., Hiratsuka-shi, Kanagawa (JP); Hiramoto, Tadahiro, TAKASAGO INTER. CORP., Hiratsuka-shi, Kanagawa (JP); Ohta, Hideaki, TAKASAGO INTER. CORP., Hiratsuka-shi, Kanagawa (JP)
(74) Representative: Uchida, Kenji

(56) References cited:
- WO-A-99/09143
- US-A- 5 804 170
- NEGISHI ET AL: "Effect of polyphenol oxidase on deodorisation" BIOSCIENCE BIOTECHNOLOGY BIOCHEMISTRY,JP,JAPAN SOC. FOR BIOSCIENCE, BIOTECHNOLOGY AND AGROCHEM. TOKYO, vol. 61, no. 12, 1997, pages 2080-2084, XP002122367 ISSN: 0916-8451

## Description

The present invention relates to deodorant compositions prepared from plant extracts or their powders. The deodorant composition of the invention can reduce or eliminate odors emanating from excrements produced by pets and other domestic animals such as dogs and cats, or from wastes of human origin, such as urine, stool and menstruation. The invention further concerns sanitary articles comprising the deodorant composition. Such a sanitary article also contains a water-absorbent polymer, and can be worn or used by men or women, as well as by pet animals. In particular, the deodorant composition of the present invention contains one or a plurality of extracts chosen from the group consisting of the extracts of rosemary plants, sunflower seeds, raw coffee grains, grape rinds, grape seeds, apple, carrot leaves and tea leaves, on the one hand, and extracts containing enzymes which oxidize at least one phenol-based compound, on the other. The sanitary article of the invention further comprises a water-absorbent polymer. The sanitary article thus manufactured is well adapted to both human and animal use.

The sanitary articles of the invention may be applied to a commonly known usage such as diapers for babies. The inventive sanitary article may also comprise hygienic bands, by incorporating appropriate pads thereinto.

Moreover, in industrially developed countries such as Japan, the proportion of aged people, relative to the entire population, has been increasing very rapidly, and is starting to create a so-called "high-age society". With the increase in the number of aged people comes the increase in sickly or bedridden elderly people needing care in their day-to-day life. This creates an undesirable overload on nursing staff who deal with incontinence due to old age and have to handle bodily wastes of bedridden people. The situation is now such that caring after the elderly has become a serious social problem. Moreover, odors emanating from these wastes afflict not only the nursing staff, but also the aged people themselves. A solution to reducing or removing odor from human wastes will therefore bring an enormous satisfaction to everybody. Hence, the need for a solution to this odor problem has been keenly felt in recent years.

On a separate account, pet animals such as dogs and cats are raised indoors more and more, compared to the past. They have thus more occasions to come into contact with people. When pet animals are raised indoors, their waste odors also raise problems. This is particularly true when their toilets are located indoors. To reduce or remove these odors, deodorizers or deodorants are usually sprayed on or around the waste. However, these measures only bring a temporary solution. Moreover, the deodorizing effect of these means is hardly satisfactory. Further, hygienic products for pets and domestic animals, such as paper diapers or toilet sheets have already been commercialized with an aim at reducing waste odors or body smell. However, no efficient means for reducing these waste odors has yet been proposed, i.e. none of the known means produces satisfactory deodorizing effects.

In the past, natural deodorant products such as plant extracts or their powders have already been used for deodorization purposes. These extracts are obtained by water extraction or hydrophilic solvent extraction. Such extracts or their powders, however, create problems of physical compatibility when they are used in combination with paper diapers or other hygienic products which contain water-absorbent polymers.

As a natural deodorant product for reducing waste, urine or menstruation odors, tea leaf extracts are described as a deodorant component in Japanese patent application published under No. HEI 10-120574. However, this deodorant product is confined to internal corporal use or an oral administration.

Furthermore, as regards sanitary articles e.g. paper diapers, there exist no commercialized articles containing a natural deodorant mixture comprised of plant extracts or their powders.

The present invention thus proposes a natural deodorant composition comprising at least one plant extract having a high deodorizing effect. The invention further concerns sanitary articles for people or for pets and animals, which can be prepared very easily in a simple way.

The inventors had already proposed a deodorant composition which contains one or a plurality of extracts chosen from the group consisting of the extracts of rosemary plants, sunflower seeds, raw coffee grains, grape rinds, grape seeds, apple and tea leaves on the one hand, and an enzyme oxidizing phenol-based compounds on the other (described in Japanese patent application, published under No. HEI 10-212221). Further studies have shown that carrot leaf extracts have a similar deodorant effect (described in Japanese patent application No. HEI 10-253173, published under No. 00-50814). The present invention is based on the discovery that the above-mentioned deodorant composition drastically reduces or removes the odors emanating from excreta or urine when combined with paper diapers or waste-disposing sheets used for people or pet animals.

It is therefore an object of the present invention to provide a deodorizing sanitary article to which is applied a water-containing substance, comprising:
(a) at least one plant extract containing at least one phenol-based compound;
(b) at least one natural enzyme extract comprising one or a group of enzymes capable of oxidizing the phenol-based compound(s); and
(c) a water-absorbent material.

The at least one extract containing the phenol-based compound(s) is chosen from the group consisting of the extracts of rosemary plants, sunflower seeds, raw coffee grains, grape rinds, grape seeds, apple, carrot leaves and tea leaves.

Further, the group of enzyme may comprise at least one enzyme selected from the group consisting of polyphenol oxidases, monophenol oxidases, hydrogen-peroxide forming oxidases and peroxidases.

The one or a group of enzymes preferably comprises an enzyme capable of oxidizing a phenol-based compound having at least two phenolic hydroxy groups.

The one or a group of enzymes may further comprise an enzyme capable of adding at least one phenolic hydroxy group to a phenol-based compound, thereby forming a phenol-based compound having at least two phenolic hydroxyl groups, and oxidizing the latter into products such as quinones.

The above-mentioned water-absorbent material is a water-absorbent polymer.

The total of the extracts (a) and (b), and the water-absorbent material (c) are mixed in a weight proportion of 1:10 to 2:1.

There are also provided a deodorizing paper diaper, a deodorizing sheet, a deodorizing floor sheet and a deodorizing toilet sheet, respectively comprising the deodorizing sanitary article as defined above.

These deodorizing sanitary article may be used for men or women, for babies, as well as for pets-and domestic animals.

As understood from the above, the invention provides a deodorizing sanitary article comprised of natural-substance ingredients, to which is applied a water-containing substance. The sanitary article comprises:
(a) at least one plant extract chosen from the group consisting of the extracts of rosemary plants, sunflower seeds, raw coffee grains, grape rinds, grape seeds, apple, carrot leaves and tea leaves;
(b) at least one natural enzyme extract comprising one or a group of enzymes capable of oxidizing at least one phenol-based compound; and
(c) a water-absorbent polymer.

The natural enzyme extract comprising one or a group of enzymes capable of oxidizing at least one phenol-based compound is selected from the group consisting of the preparations of burdock plants, pears, apples, bananas, potatoes, peaches, taros, grapes, lotus roots and mushrooms.

The present invention therefore provides a sanitary product for people or pet animals, characterized by comprising a deodorant composition which contains one or a plurality of extracts chosen from the group consisting of the extracts of rosemary plants, sunflower seeds, raw coffee grains, grape rinds, grape seeds, apple, carrot leaves and tea leaves, on the one hand, and extracts containing enzyme(s) oxidizing at least one phenol-based compound, on the other, the sanitary products further comprising water-absorbent polymers.

The above and the other objects, features and advantages of the present invention will be made apparent from the following description of the preferred embodiments, given as non-limiting examples.

The deodorant composition used in the present invention contains one or a plurality of extracts chosen from the group consisting of the extracts of rosemary plants, sunflower seeds, raw coffee grains grace rinds, grape seeds, apple, carrot leaves and tea leaves on the one hand, and extracts containing enzyme(s) capable of oxidizing at least one phenol-based compound on the other. The deodorant composition described above comprises extracts obtained from the plants supra which exhibit a strong deodorizing effect in the presence of water. Further, such a deodorant composition retains its deodorizing effects for a long time. When there is no water, the composition shows no deodorizing activities, and is quite stable. Under normal storage conditions, the composition can retain its deodorizing activities over more than a year.

One component of the deodorant composition supra is one or a plurality of plant extracts chosen from the group consisting of the extracts of rosemary plants, sunflower seeds, raw coffee grains, grape rinds, grape seeds, apple, carrot leaves and tea leaves. These extracts may include any extract which contains at least one phenol-based compound, extractable by water, an alcohol, an organic solvent or a mixture thereof.

Extraction solvents are chosen appropriately, depending on the intended use of the deodorant compositions. Examples of such extraction solvents include water, ethanol, propanol, a butanol, acetone, hexane, propyleneglycol, water-containing ethanol and water-containing propyleneglycol. However, hot water, water-containing ethanol or water-containing propyleneglycol is preferably used.

The extraction is conducted under usual extraction conditions. The extracts obtained are concentrated in vacuo. The concentrates are then dried by spray-drying, lyophilization or the like, so as to obtain dried powder.

Another component of the deodorant composition according to the present invention comprises extracts containing one or a group of enzymes capable of oxidizing at least one phenol-based compound. Preferably, these enzymes include at least one enzyme that can transform the phenol-based compound(s) into quinone-based compound(s) through oxidation, and /or include at least one enzyme that can perform the foregoing oxidation, while adding at least one hydroxy group to the phenol-based compound(s) and oxidizing it or them into quinone(s).

Any enzyme having the above functions can be used for the purpose of the invention. Examples of such enzymes are a polyphenol oxidase, a catechol oxidase, a monophenol oxidase, a hydrogen-peroxide forming oxidase and a peroxidase. However, laccases, tyrosinases, glucose oxidases and peroxidases are preferably used.

The examples of enzymes capable of oxidizing the phenol-based compound(s) while adding at least one hydroxy group thereto may include monophenol oxidases such as phenolases, tyrosinases and cresolases.

One or a mixture of enzymes extracted from burdock plants or from pears, apples, bananas, potatoes, peaches, taros, grapes, lotus roots or mushrooms are used.

The examples of the phenol-based compounds are described in US patent 5,804,170.

The deodorant composition according to the invention may be used as powder or granules, depending on the intended use. Such powder or granules may include a carrier component comprising saccharides such as dextrins, cyclodextrins, glucose, lactose and different types of starch. They may also include a filler component comprising inorganic salts such as anhydrous sodium sulfate, anhydrous sodium silicate and sodium chloride, or other compounds such as sodium polyacrylate. A suitable carrier compound may be chosen from the corresponding carrier group, whereas a suitable filler compound may be chosen from the corresponding filler group. They are then added into the deodorant composition and mixed.

A water-absorbent polymer is used as water-absorbent material of the present invention. Paper diapers containing the foregoing deodorant composition may thus be manufactured. Commercial paper diapers usually contain not only a polyacrylate-salt type compound, but also a water-absorbent polymer such as a starch-polyacrylate salt graft compound, a polyvinyl-alcohol type compound, a copolymer of vinyl alcohol and acrylate salts, and a copolymer of isobutylene and maleic acid, so that the liquid portion of the wastes is absorbed. A water-absorbent polymer such as described above may be used for the purpose of the present invention. Such a polymer may also be used in bed sheets or bed mats for bedridden aged people, or in incontinence pads and sanitary napkins, or in paper diapers, toilet sheets and floor sheets for pet animals.

However, use of the deodorant composition according to the invention is not particularly limited by the type of water-absorbent polymer employed. An appropriate combination is chosen such that enzyme activities in the deodorant composition are not impeded. According to the invention, the deodorant composition and the water-absorbent polymer are mixed in a weight proportion of 1: 10 to 2:1.

In the manufacture of the sanitary articles according to the invention, the above-mentioned deodorant composition can be incorporated as such. However, when a water-absorbent polymer is used, as in case of paper diapers, the deodorant composition may be mixed preliminarily with the water-absorbent polymer, and homogenized therein prior to its use.

The amount of deodorant composition used may depend on circumstances. When used in paper diapers, 0. 05 to 4. 0 g of deodorant composition are used per gram of water-absorbent polymer. When used in adult paper diapers, 0. 10 to 1. 00 g of deodorant composition is used per gram of water-absorbent polymer. When used in baby or infant paper diapers, 0. 05 to 0. 30 g of deodorant composition is used per gram of water-absorbent polymer. In the case of paper diapers for pets, 0. 10 to 1. 00 g of deodorant composition is used per gram of water-absorbent polymer.

When less than 0. 01 g of deodorant composition is used per gram of water-absorbent polymer, sufficient deodorizing effect cannot be obtained for any intended use. Conversely, when the amount of deodorant composition exceeds 5. 0 g per gram of water-absorbent polymer, the base material emits a strong odor of plant extracts or oxidation enzymes. As a result, another kind of odor problem may be raised, and the initial deodorization object may not be attained.

In addition to the paper diapers mentioned above, the composition of the present invention can be used in other sanitary articles, e.g. bed sheets or bed mats intended for sickly aged people, incontinence pads, sanitary napkins or the like. When used in such articles, the deodorant composition can remove or reduce odors emitted from wastes and urine efficiently. The deodorant composition can also be used in paper diapers for waste handling, for pet animals such as dogs or cats raised indoors. The deodorant composition may also be applied to floor sheets or toilet cover sheets used for hygienic purposes. In all the above uses, the same deodorizing effect can be obtained.

The sanitary articles for people and pet animals according to the invention can be prepared quickly and easily. They thus have a practical utility, but nonetheless exhibit a surprising effect for odor removal or odor reduction.

### Examples

The invention is described hereinafter in detail with reference to Test Examples and Preparation Examples.

### Preparation Example 1

### Preparation of a deodorant composition

### (1) Preparation of raw coffee grain extracts

1 kg of raw coffee grains is ground by a grinder or mill, to yield powder having a mesh of 5 mm. The powder is supplemented with 10 liter of water, and extracted therewith for 2 hours at 85 to 95 °C. After the extraction, the liquid fraction is filtered, and the resultant filtrate is condensed and dried, thereby obtaining 182 g of raw coffee grain extracts.

### (2) Production of burdock plant preparations (including burdock enzyme)

100 g of burdock, and 400 ml of acetone cooled to - 20 °C, are put into a mixer, and triturated therein. The mixture is then filtered under aspiration, to yield a residue and a filtrate. The residue obtained is washed with 500 ml of acetone-water solution (80 % acetone) at 5 °C. Acetone is then distilled away from the residue. Subsequently, the residue is dried by lyophilization, to yield 20 g of burdock powder preparations.

The raw coffee grain extracts obtained by preparation process (1) and the burdock powder preparations obtained by production process (2) are mixed in a weight proportion of 1 : 1, whereby a deodorant composition is obtained.

### Test Example 1

### Method

A commercially available paper diaper for pets, manufactured by Tokyo Pet Kabushiki Kaisha under the trade name "Happy Jelly Sheet" includes a water-absorbent polymer. About one gram of this polymer is removed from the above paper diaper, mixed with 0. 5 g of deodorant composition obtained by Preparation Example 1, and reintegrated into the same diaper. The paper diaper thus prepared is worn by a small Shetland dog. After urination, the paper diaper is removed, and odors arising therefrom are evaluated by five panelists through smelling. A paper diaper containing no deodorant composition is used as comparative example.

### Results

Results of the evaluation tests are shown in Table 1, in which :
- 1 :: No urine odor is smelt ;
- 2: : Slight urine odor is smelt ;
- 3 :: weak urine odor is smelt ;
- 4 :: Urine odor is smelt ;
- 5 :: Strong urine odor is smelt ;
- 6 :: Very strong urine odor is smelt.

**TABLE 1:**

| Odor Intensity Score (6-level evaluation) | | | | | | |
|---|---|---|---|---|---|---|
| Sample | Panelist A | Panelist B | Panelist C | Panelist D | Panelist E | Average |
| Inventive article | 1.0 | 1.0 | 1.0 | 1.5 | 1.5 | 1.2 |
| Comparative article | 4.0 | 3.0 | 3.0 | 5.5 | 5.5 | 4.2 |

As shown in Table 1, when the inventive paper diaper containing the deodorant composition is used, three panelists among five smelt no odor, indicating that the urine odor has been nearly completely removed. By comparison, when the paper diaper containing no deodorant composition is used, all panelists smelt a urine odor. Two among them, in particular, have judged that the urine odor was strong.

### Test Example 2

### Method

A commercially available paper diaper for adult use, manufactured by Unicharm Kabushiki Kaisha under the trade name "Mamy Poko L-size" includes a water-absorbent polymer. About one gram of this water-absorbent polymer is removed from the above paper diaper, mixed with 0. 2 g of deodorant composition obtained by Preparation Example 1, and reintegrated into the same diaper. The paper diaper thus prepared is used to absorb 100 ml of adult male urine. After 5 minutes, the paper diaper is subjected to five panelists for smelling tests. For comparison, there are provided a same paper diaper containing no deodorant composition (comparative article), and two kinds of commercial paper diaper containing a known deodorant (commercial articles 1 and 2).

### Results

Results of the smelling tests are shown in Table 2, in which odor is evaluated and classified into six levels as in the case of Test Example 1.

**TABLE 2:**

| Odor intensity Score (6-level evaluation) | | | | | | |
|---|---|---|---|---|---|---|
| Sample | Panelist A | Panelist B | Panelist C | Panelist D | Panelist E | Average |
| Inventive article | 1.0 | 1.0 | 1.0 | 1.0 | 1.0 | 1.0 |
| Comparative article | 5. 0 | 5. 0 | 5. 0 | 5. 0 | 5. 0 | 5. 0 |
| Commercial article 1 | 5.0 | 5.0 | 5.0 | 4.5 | 5.5 | 5.0 |
| Commercial article 2 | 5.0 | 5.0 | 5.0 | 5.0 | 6.0 | 5.2 |

As shown in Table 2, when commercial or comparative articles are used, all panelists have smelt a strong urine odor, indicating that those articles have a poor deodorizing effect. By contrast, when the inventive paper diaper is used, deodorization is so efficient that no panelist detected a urine odor.

### Test Example 3

### Method

A urine-absorbent pad is tested with a bedridden elderly person who employs paper diapers in his daily life at home. A urine-absorbent pad (incontinence pad) used in combination with a paper diaper manufactured by Kabushiki Kaisha Crecia under the trade name "Acti Nyô Tori Pad Regular" includes a water-absorbent polymer. About one gram of this polymer is removed from the urine-absorbent pad, mixed with 0. 20 g of deodorant composition of Preparation Example 1, and reintegrated into the original pad. This pad is then used by the elderly person.

### Results

When a urine-absorbent pad is replaced after having been served with urine or stool, a strong odor usually spreads out. However, when a pad containing the deodorant composition is used, this strong odor is so drastically reduced that the odor can hardly be detected.

### Test Example 4

### Method

A care sheet is tested with a bedridden elderly person who uses this sheet in his daily life at home. Such a care sheet, manufactured by Hakujûji Kabushiki Kaisha under the trade name "Salba Care Sheet, mesh-type for half the body" includes a water-absorbent polymer. About one gram of this polymer is removed from the sheet, mixed with 2. 0 g of deodorant composition of Preparation Example 1 per sheet, and reintegrated into the original sheet. This sheet is then laid out around the position where waste will collect, and further covered with a common sheet. The elderly person passes a night thereon. Next day, the common sheet is removed, and the odor intensity is tested by smelling. For comparison, a same care sheet, but containing no deodorant composition of the present invention, is used in another day.

### Results

Table 3 shows the results obtained after 6-level evaluation having been effected, as in the case of Test Example 1.

**TABLE 3 :**

| Odor Intensity Score (6-level evaluation) | | | | | | |
|---|---|---|---|---|---|---|
| Sample | Panelist A | Panelist B | Panelist C | Panelist D | Panelist E | Average |
| Inventive article | 1.0 | 1.5 | 1.0 | 1.0 | 1.0 | 1.1 |
| Comparative article | 5.0 | 4.0 | 6.0 | 6.0 | 5.0 | 5.2 |

A strong odor is normally smelt when changing a sheet after being served with urine or stool. According to the tests using a sheet including the deodorant composition of the present invention, this strong odor is so completely reduced that it can hardly be detected.

### Preparation Example 2 (time stability)

A deodorant composition of Preparation Example 1 is kept in a brown-colored glass bottle for one year, and the deodorizing effect of the composition is measured with the passage of time.

### Measuring Method

20 mg of deodorant composition and 1. 5 ml of distilled water are put into a vial having a volume of 100 ml, and an odor-emitting compound such as ammonia, trimethylamine and methylmercaptan is added thereto, so as to give an initial concentration of 200 ppm. After manually shaking for 10 minutes, 50 ml of gas contained in the vial is sampled into a detector tube, manufactured by Gastech Kabushiki Kaisha. There is then measured the concentrations of odor-emitting compounds that remain in the gas.

### Results

The results obtained are shown in Table 4.

**TABLE 4**

| | | Deodorizing effect at the start | Deodorizing effect after 1 year |
|---|---|---|---|
| Odor-emitting gas | Initial concentration | Measure (deodorization rate) | Measure (deodorization rate) |
| Ammonia | 200 ppm | 0 ppm (100 %) | 10 ppm (95 %) |
| Trimethylamine | 200 ppm | 0 ppm (100 %) | 10 ppm (95 %) |
| Methylmercaptan | 200 ppm | 0 ppm (100 %) | 0 ppm (100 %) |

### Preparation Example 3

### Method

Urine of adult men is first collected. Immediately after its collection, 0. 01 to 0. 30 g of deodorant composition of Preparation Example 1 is added stepwise to 100 ml of the urine. The resultant mixture is subjected to five panelists for smelling tests. For comparison, a urine solution containing no deodorant composition of the present invention is prepared in the same way.

### Results

Results of the smelling tests are shown in Table 5, in which 6-level evaluation is effected as in the case of Test Example 1.

**TABLE 5 :**

| Odor Intensity Score (6-level evaluation) | | | | | | |
|---|---|---|---|---|---|---|
| amount Added | Panelist A | Panelist B | Panelist C | Panelist D | Panelist E | Average |
| Blank | 6.0 | 6.0 | 6.0 | 6.0 | 6.0 | 6.0 |
| 0.01 g | 6.0 | 6.0 | 6.0 | 6.0 | 6.0 | 6.0 |
| 0.03 g | 4.0 | 5.0 | 4.0 | 4.5 | 5.0 | 4.5 |
| 0.05 g | 3.0 | 4.0 | 3.0 | 3.0 | 4.0 | 3.5 |
| 0.08 g | 2.0 | 4.0 | 3.0 | 3.0 | 3.0 | 3.0 |
| 0.10 g | 1.0 | 2.5 | 1.5 | 2.0 | 3.0 | 2.0 |
| 0.20 g | 1.0 | 1.0 | 1.0 | 1.0 | 1.0 | 1.0 |
| 0.30 g | 1.0 | 1.0 | 1.0 | 1.0 | 1.0 | 1.0 |

As shown in Table 5, when 0. 10 g of deodorant composition is added to 100 ml of adult men's urine, urine odor is only faintly smelt. When the amount of the deodorant composition exceeds 0. 20 g, urine odor is completely removed. An addition of only 0. 08 g of deodorant composition already reduces urine odor to a half of the blank sample.

In the present invention, there is provided a mixture of one or a plurality of extracts chosen from the group consisting of the extracts of rosemary plants, sunflower seeds, raw coffee grains, grape rinds, grape seeds, apple, carrot leaves and tea leaves, and extracts containing one or a group of enzymes capable of oxidizing at least one phenol-based compound, as well as a water-absorbent polymer. When this mixture is used in sanitary articles such as paper diapers for persons or pet animals, it can drastically reduce or completely remove the odors emanating from human or animal wastes. Moreover, the deodorant compositions of the invention can be prepared very easily and economically, and thus applied to varieties of sanitary articles.

## Claims

1. A deodorizing sanitary article comprised of natural-substance ingredients, to which is applied a water-containing substance, said sanitary article comprising:
(a) at least one plant extract selected from the group consisting of the extracts of rosemary plants, sunflower seeds, raw coffee grains, grape rinds, grape seeds, apple, carrot leaves and tea leaves;
(b) at least one natural enzyme extract, comprising one or a group of enzymes capable of oxidizing at least one phenol-based compound, selected from the group consisting of the preparations of burdock plants, pears, apples, bananas, potatoes, peaches, taros, grapes, lotus roots, and mushroom; and
(c) a water-absorbent polymer;
the total of both extracts (a) and (b), and said water-absorbent polymer (c) being mixed in a weight proportion of 1:10 to 2:1.

2. A deodorizing sanitary article according to claim 1, wherein said at least one plant extract (a) is a raw coffee grain extract and said at least one natural enzyme extract (b) is a burdock plant preparation.

3. A deodorizing paper diaper comprising the deodorizing sanitary article defined in claim 1 or 2.

4. A deodorizing sheet comprising the deodorizing sanitary article defined in claim 1 or 2.

5. A deodorizing floor sheet comprising the deodorizing sanitary article defined in claim 1, 2 or 4.

6. A deodorizing toilet sheet comprising the deodorizing sanitary article defined in claim 1, 2 or 4.

7. Use of the deodorizing sanitary article according to claim 1 or 2 for men or women.

8. Use of the deodorizing sanitary article according to claim 1 or 2 for babies.

9. Use of the deodorizing sanitary article according to claim 1 or 2 for pets-and domestic animals.

## Patentansprüche

1. Desodorierender Hygienegegenstand, umfassend Bestandteile natürlicher Substanzen, zu welchem eine Wasser-enthaltende Substanz zugefügt wird, wobei der Hygienegegenstand umfaßt:
(a) mindestens einen Pflanzenextrakt, ausgewählt aus der Gruppe, bestehend aus den Extrakten von Rosmarinpflanzen, Sonnenblumenkernen, Rohkaffeekörnern, Weintraubenschalen, Weintraubenkernen, Äpfel, Karottenblättern und Teeblättern;
(b) mindestens einen natürlichen Enzymextrakt, umfassend ein oder eine Gruppe von Enzymen, befähigt zur Oxidation mindestens einer Verbindung auf Phenol-Basis, ausgewählt aus der Gruppe, bestehend aus den Formulierungen von Klettenpflanzen, Birnen, Äpfeln, Bananen, Kartoffeln, Pfirsichen, Wasserbrotwurzeln, Weintrauben, Lotuswurzeln und Pilzen, und
(c) ein Wasser-absorbierendes Polymer;
wobei die Gesamtheit beider Extrakte (a) und (b) und das Wasserabsorbierende Polymer (c) in einem Gewichtsverhältnis von 1:10 bis 2:1 gemischt sind.

2. Desodorierender Hygienegegenstand nach Anspruch 1, wobei der mindestens eine Pflanzenextrakt (a) ein Extrakt von Rohkaffeekörnern ist und der mindestens eine natürliche Enzymextrakt (b) eine Klettenpflanzenformulierung ist.

3. Desodorierende Papierwindel, umfassend den desodorierenden Hygienegegenstand, wie in Anspruch 1 oder 2 definiert.

4. Desodorierende Lage, umfassend den desodorierenden Hygienegegenstand, wie in Anspruch 1 oder 2 definiert.

5. Desodorierende Bodenlage, umfassend den desodorierenden Hygienegegenstand, wie in Anspruch 1, 2 oder 4 definiert.

6. Desodorierende Toilettenlage, umfassend den dosodorierenden Hygienegegenstand, wie in Anspruch 1, 2 oder 4 definiert.

7. Verwendung des desodorierenden Hygienegegenstands gemäß Anspruch 1 oder 2 für Männer oder Frauen.

8. Verwendung des desodorierenden Hygienegegenstands gemäß Anspruch 1 oder 2 für Babys.

9. Verwendung des desodorierenden Hygienegegenstands gemäß Anspruch 1 oder 2 für Streichel- und Haustiere.

## Revendications

1. Article hygiénique déodorisant comprenant des ingrédients de substances naturelles, auxquels est appliquée une substance contenant de l'eau, le dit article hygiénique comprenant :
(a) au moins un extrait de plante choisi dans le groupe composé des extraits de romarin, graines de tournesol, grains de café brut, peaux de raisin, pépins de raisin, pommes, feuilles de carotte et feuilles de thé ;
(b) au moins un extrait d'enzyme naturelle, comprenant une enzyme ou un groupe d'enzymes pouvant oxyder au moins un composé à base de phénol, choisie dans le groupe comprenant les préparations de bardane, poires, pommes, bananes, pommes de terre, pêches, taros, raisins, racines de lotus et champignons ;et
(c) un polymère absorbant l'eau ;
le total des deux extraits (a) et (b) et du dit polymère absorbant l'eau (c) étant mélangé dans une proportion de 1:10 à 2:1 en poids.

2. Article hygiénique déodorisant selon la revendication 1, dans lequel le dit au moins un extrait de plante (a) est un extrait de grains de café brut et le dit au moins un extrait d'enzyme naturelle (b) est une préparation de bardane à l'état de plante.

3. Couche en papier déodorisante comprenant l'article hygiénique déodorisant selon la revendication 1 ou 2.

4. Feuille déodorisante comprenant l'article hygiénique déodorisant selon la revendication 1 ou 2.

5. Feuille de sol déodorisante comprenant l'article hygiénique déodorisant selon la revendication 1, 2 ou 4.

6. Papier hygiénique déodorisant comprenant l'article hygiénique déodorisant selon la revendication 1, 2 ou 4.

7. Utilisation de l'article hygiénique déodorisant selon la revendication 1 ou 2, pour homme ou femme.

8. Utilisation de l'article hygiénique déodorisant selon la revendication 1 ou 2, pour enfants.

9. Utilisation de l'article hygiénique déodorisant selon la revendication 1 ou 2, pour animaux de compagnie et animaux domestiques.
